# EUROPEAN PATENT SPECIFICATION

(11) **EP 4 161 448 B1**
(45) Date of publication and mention of the grant of the patent: **10.07.2024**
(21) Application number: 21735395.2
(22) Date of filing: 31.05.2021
(51) Int. Cl.: A61F 2/38

(54) **UNICOMPARTIMENTAL KNEE PROSTHESIS**
EINTEILIGE KNIEPROTHESE
PROTHÈSE DE GENOU UNI-COMPARTMENTALES

(30) Priority: 08.06.2020 IT 202000013543
(43) Date of publication of application: 12.04.2023
(73) Proprietor: GRUPPO BIOIMPIANTI S.R.L., 20068 Peschiera Borromeo (MI) (IT)
(72) Inventor: PIAVANI, Alberto Giulio, 24050 Bariano (BG) (IT); CUSMA' DOVICO GUATTERI, Gianluca, Diyar al Muharraq, Kingdom of Bahrain (BH)
(74) Representative: Savi, Massimiliano
(86) International application number: PCT/IB2021/054765
(87) International publication number: WO 2021/250504

(56) References cited:
- WO-A1-2011/028624
- WO-A1-2013/131066
- WO-A1-2017/060221
- US-A1- 2010 305 575
- US-A1- 2012 116 524
- US-A1- 2016 228 255

## Description

### FIELD OF THE INVENTION

The present invention relates to the technical field of surgical prosthesis, in particular to the field of unicompartmental knee prostheses.

### BACKGROUND ART

Unicompartmental arthroplasty operations (*Unicondylar Knee Arthroplasty* - UKA) represent a solution which has now become consolidated in the treatment of pathologies affecting only one compartment of the knee In fact, the use of unicompartmental femoral-tibial knee arthroplasty constitutes a valid alternative to total knee replacement operations in the case where one of the two compartments of the knee is not in a pathological state and in the case where both the cruciate ligaments of the knee are intact and functional.

In general, with respect to a Total Knee Arthroplasty - TKA, the UKA operation is less invasive, characterized by lower blood loss and fewer complications, and allows quicker recovery of the patient. Despite the efforts to simplify the surgical technique for the unicompartmental arthroplasty implant, such surgical operation is still complex and problematic.

In general, technical literature reports that the UKA procedure allows the articular kinematics to be restored in a satisfactory way, thus producing better joint functionality at a lower cost with respect to TKA operations. In fact, it can be stated that the maintenance of the anterior cruciate ligament and its mechanoreceptors allows more similar joint kinematics during bending to the kinematics of a physiological joint, as demonstrated by various relevant studies. These comparison studies between TKA and UKA operations report better functional recovery and greater proprioception in the case of UKA.

Despite the excellent functional results reported, the revision rate that can be observed in UKA operations still remains rather high even if the revision risk for infection is considerably lower with respect to TKA type operations.

The reasons for revision include the fact that, as only one of the two compartments has been replaced, the pathology can progress in the other healthy compartment too, requiring a revision of the UKA in TKA. Furthermore, the unicompartmental knee replacement at lateral compartment level is more complicated with respect to the medial one because of anatomical differences. The lateral ligament is slacker than the medial one, and the rear movement of the lateral condyle is wider than that of the medial condyle. For these reasons, the clinical results of lateral UKA operations reported are often poorer than medial ones. To date, lateral procedures represent about 5-10% of all unicompartmental procedures.

In general, careful selection of patients and excellent knowledge of the arthroplasty and of the surgical technique appears necessary in order to obtain optimal results and long-term survival of the unicompartmental implant.

In relation to pathologies that require this type of surgical operation, osteoarthritis is by far the main diagnosis for which primary unicompartmental knee replacement procedures are performed. Patients most commonly subject to this surgical procedure are males, aged between 55 and 74.

The state of the art offers a wide variety of types of unicompartmental knee prosthesis (UKP) implants in relation to the type of fixation or the type of implant: articular implants, the tibial component and the femoral one.

In relation to fixation, femoral and tibial components are available for fixation with bone cement (cemented) or for biological fixation (titanium and/or hydroxyapatite coating).

Unlike the total knee arthroplasty which also provides for hybrid fixation (cementless femur and cemented tibia) in a percentage equal to about 10% of cases, in unicompartmental knee arthroplasty, hybrid fixation is very rarely used in clinical practice, amounting to about 1% of total cases.

The most commonly used type of fixation is the cemented one. When cementless components are used, in particular with a fixed implant, the implant can be completed with a cancellous bone screw for the tibial component. In accordance with this trend, all of the companies that manufacture prosthetics for this type of operation have versions to be cemented in their catalogs whereas there are fewer of those which also offer cementless prosthesis versions.

In relation to articular inserts: mobile or fixed articular inserts are available.

Mobile articular inserts are relatively free to move on the tibial component, guided by the femoral congruence. Mobile inserts are adapted to restore the articular movement and to limit the phenomenon of wear; they are accompanied by an equal complex operating technique based on perfect ligament balance and a higher risk of failure in the short term (insert dislocation).

Fixed articular inserts are adapted to be fitted onto the tibial component or onto a tibial component entirely made of polyethylene; fixed inserts are not subject to the risk of dislocation and therefore can compensate better for any operating techniques or clinical conditions that are not ideal. Unlike mobile inserts, which can exploit maximum congruence between the femur and the insert, to guarantee mobility, fixed inserts must work at very low congruence values and are therefore subject to higher wear values (point or line contact).

The version most commonly used in the world is the fixed insert one.

All the companies that manufacture prosthetics for this type of operation propose a prosthesis version with a fixed insert whereas there is a smaller number of those that also offer the prosthesis version with a mobile insert.

In relation to the tibial component, for both prostheses with a fixed insert and with a mobile insert, tibial components formed by a polyethylene insert and a metal back are available. For prostheses with a fixed insert, tibial components comprising a single piece of polyethylene (all poly) are also available.

The advantage of the metal back version is the resistance offered by the metal back, especially considering the limited area of the UKP; the advantage of the all poly version is the larger volumetric quantity of polyethylene and therefore the improved behavior towards wear.

The version most commonly used in the world is the metal back one.

The materials used for the metal back are titanium or cobalt chromium alloy.

All the companies that manufacture prostheses for this type of operation offer metal back versions, whereas there is a smaller number of those which also offer the all poly version.

The femoral component is the one that more greatly allows the implant to be adapted to the patient's specific conditions rather than adapting the patient to the implant, as happens for the other components.

Three very distinct types of femoral component can be distinguished, each with a series of advantages and disadvantages recognized in literature and clinical practice:
The femoral component with a spherical portion is mainly used for mobile inserts. These femoral components are characterized by their total congruence with the mobile polyethylene insert in order to limit articular stress and the possibility of dislocation, which are still possible with this type of insert. Their shape is at the same time the reason for their clinical success but also for their main weaknesses: the complex, intramedullary operating technique, and lack of adaptability to the individual patient's femoral condyle. Following the implantation of this type of femoral components, "overhang" cases can arise, i.e. projections extending beyond the natural femoral condyle.

The femoral coating component, also known as the resurfacing component, is a widespread type especially for European manufacturers.

These femoral components are characterized by the reduced thickness of the femoral component and therefore they have reduced invasiveness (between 2 mm and 4 mm); in fact, at distal level they only require the removal of the articular cartilage without removing any bone volume. If on one hand this characteristic allows a great adaptability to the patient's femoral condyle, on the other hand it implies a poorly guided operating technique, in which the operating surgeon is required to have excellent manual dexterity. Furthermore, in case of unexpected problems at the femoral level (e.g. necrosis), the further removal of femoral bone is not possible except to the detriment of altering the joint line and therefore the possibility of hypercorrection of the mechanical axis.

In general, users of this technique are surgeons used to a large number of annual UKP operations, a necessary element for maintaining manual dexterity in this type of procedure; less experienced users may find themselves in difficulty with this technique, both in general and, especially, in the event of unexpected circumstances.

The femoral component with resections is a common type especially for US manufacturers.

It is characterized by distal, posterior and oblique femoral resections which naturally require a femoral component with a greater thickness with respect to that required in the case of resurfacing femoral components. This detail is not very popular with surgeons performing mini-invasive operations as this greater invasiveness (usually at least 7 mm) is closer to that of a total prosthesis (usually about 9 or 10 mm) with respect to that of resurfacing (from 2 to 4 mm). Furthermore, the larger dimensions of the femoral component with resections, especially with respect to lateral condyles (in particular hypoplasic) can result in excessive dimensions and not permit the use thereof.

The operating technique relating to femoral components with resections is however largely guided by relevant resection blocks and is therefore popular with surgeons with less operating experience.

The version of femoral component most commonly used in the world is the one with resections.

Patent documents WO/2013/131066, US2012/0116524, US2010/0305575 and SG10201405092SA describe examples of state-of-the-art articular repair and replacement implants for knee joints.

Considering the above, it is clear that unicompartmental knee arthroplasty (UKP) prostheses are very different and distant from one another both from a functional point of view and from the point of view of the compatibility of the articular insert to be combined. A reflection of this profound diversity is in some way testified by the fact that the manufacturers of this type of prosthesis in general only propose one version of the femoral component.

In light of all this, it is not therefore possible, especially for surgeons with a large amount of experience, to adapt the prosthetic model to the individual patient. In particular, in the event of adaptations on the femoral joint line, only some prostheses with resections allow the height of the femoral distal cut to be adjusted through appropriate spacers, and therefore through the implant of a component with a notable thickness; from this point of view it is not permitted, for example, to choose between a prosthesis with a greater thickness and one with a lesser thickness at femoral level.

Therefore, it is clear that there is a need for a new design of unicompartmental knee prosthesis, capable of solving the problems of the prostheses in the state of the art.

### SUMMARY OF THE INVENTION

The object of the present invention comprises a modular prosthesis intended for femoral-tibial unicompartmental knee arthroplasty with a fixed insert. Said prosthesis has original solutions adapted to allow the surgeon to adapt the most correct model to the patient and therefore offer a greater degree of flexibility and the possibility of better implant performance.

The prosthesis according to the present description comprises a femoral component which can preferably be made of various materials and anatomical measurements and in two variants, both having a thickness less than 7 mm and at least one fastening pin; a tibial component, which can also preferably be made in two variants and of various materials and anatomical measurements and an articular insert which can preferably be made in only one variant, also of various materials and anatomical measurements. The aforesaid parts are usable in different combinations thus giving the modular prosthesis according to the present description unprecedented flexibility of use and adaptability to every type of patient.

The two variants of said femoral component are indicated below, for convenience purposes with the initials 3CUT and RES.

The femoral component of the modular prosthesis according to the invention known as RES, provides for the sole removal of cartilage at distal level and the replacement thereof with a femoral shield. This version is made of metallic material (e.g. CrCoMo alloy) and comprises only one fastening pin Advantageously a reinforced anti-rotational flap can be provided. It is the most conservative version of the two as it only provides for the removal of cartilage at distal level and the replacement thereof with a femoral shield preferably having a thickness around 4 mm. The thickness, from the distal to the posterior, constant and less than 7 mm, allows the invasiveness with respect to the patient's femur to be reduced to a minimum.

The femoral component of the modular prosthesis according to the invention known as 3CUT, provides for three bone resections for resting the three surfaces of the femoral component and fastening it: a distal one, a posterior one and an oblique one. This version is made of metallic material (e.g. CrCoMo alloy) and comprises two fastening pins but maintains the characteristics of reduced invasiveness by virtue of a constant thickness, from the distal to the posterior, less than 7 mm. The two preferred tibial components, according to the present description, comprise a version made of plastic material, preferably polyethylene, indicated below, for convenience purposes, with the initials AllPoly, and a version made of plastic material (polyethylene) with a metal back (e.g. made of CrCoMo or titanium alloy), indicated below, for convenience purposes, with the initials MetalBack.

In the MetalBack version there is a fixed articular insert preferably made of polyethylene.

Both the tibial components are compatible with both the femoral components and have a very low joint congruence contact surface, preferably having a double ellipse profile with greater congruence in the rear part. For example, the ellipses of said double ellipse can be chosen with a semiaxis equal to about 6 mm and 38 mm in the event of a smaller ellipse, responsible for the profile with greater congruence of the rear part, and equal to about 6 mm and 200 mm in the case of the greater ellipse, responsible for the profile with greater congruence of the front part.

This joint contact surface is formed on the upper face of the AllPoly type tibial component or on the upper face of the articular insert adapted to be engaged with the MetalBack type tibial component.

Said articular insert is preferably made in 4 different sizes, having minimum thicknesses for example equal to about 5.5 mm, 6.5 mm, 7.5 mm and 9.5 mm.

The polyethylene components of the two versions of tibial component (the entire AllPoly tibial component and the fixed articular insert for the MetalBack tibial component) may advantageously be made in the linear UHMWPE version, the XLink (cross linked) version and XLink version with added Vitamin E, especially developed and used for knee prosthetics. Vitamin E is a powerful antioxidant and has an anti-aging effect. The addition of Vitamin E therefore prevents the oxidation phenomena to which the XLink polyethylene is subject following the cross linking reaction and therefore reduces the wear thereof which, as well as being the result of the contact stress and shear strain which act between the components of the prostheses, is also caused by the oxidation of the materials themselves.

### BRIEF DESCRIPTION OF THE FIGURES

Further features and advantages of the invention will become apparent from the following detailed description provided by way of example and not by way of limitation, with the aid of the Figures shown in the accompanying drawings, in which:
Fig. 1 illustrates a preferred embodiment of the RES type femoral component according to the present description;
Fig. 2 illustrates a preferred embodiment of the 3CUT type femoral component according to the present description;
Fig. 3 illustrates a preferred embodiment of the AllPoly type tibial component according to the present description;
Fig. 4 illustrates a preferred embodiment of the MetalBlack type tibial component according to the present description, and
Fig. 5 illustrates the preferred embodiment of the tibial insert according to the present description.

The following description of exemplary embodiments refers to the accompanying drawings. The same reference numerals in the various drawings identify the same or similar elements. The following detailed description does not limit the invention. The scope of the invention is defined by the appended claims.

### DETAILED DESCRIPTION OF THE INVENTION

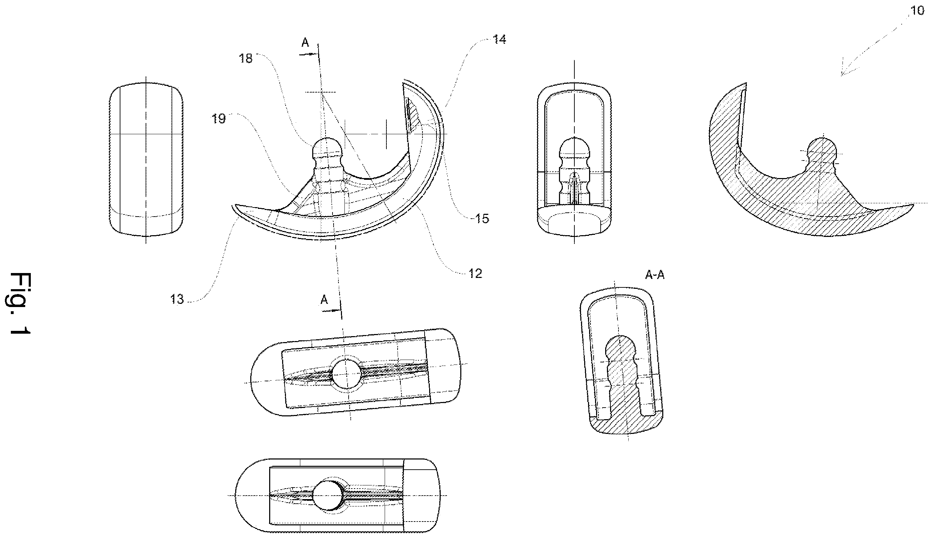

With reference to the appended Figs. 1 - 6, the femoral-tibial unicompartmental knee prosthesis with a fixed insert for knee arthroplasty according to the present description comprises a first femoral component 10 adapted to be connected to the end of a femur and a second tibial component 11 adapted to be connected to the end of a tibia.

The first femoral component 10 has a curved main body 12 with a distal end 13 and a rear end 14, a convex outer side and an inner side comprising fastening means at the end of a femur. The maximum thickness 15 of said main body is preferably less than or equal to 7 mm. In particular, the maximum thickness 15 of said main body 12 may be equal to 6.75 mm.

In a preferred embodiment, shown in Figure 1, known as RES, the first femoral component 10 has a curved main body 12 with an inner side comprising a pin 18 facing a substantially radial direction. Advantageously, said pin 18 may be associated with an anti-rotational flap 19 oriented in the longitudinal direction. In this preferred embodiment, the maximum thickness of said main body 12 is preferably less than or equal to 7 mm at said rear end 14 and less than or equal to 4.5 mm at said distal end 13. In particular, the maximum thickness of said main body 12 may be equal to 6.75 mm at said rear end 14 and may be equal to 4 mm at said distal end 13.

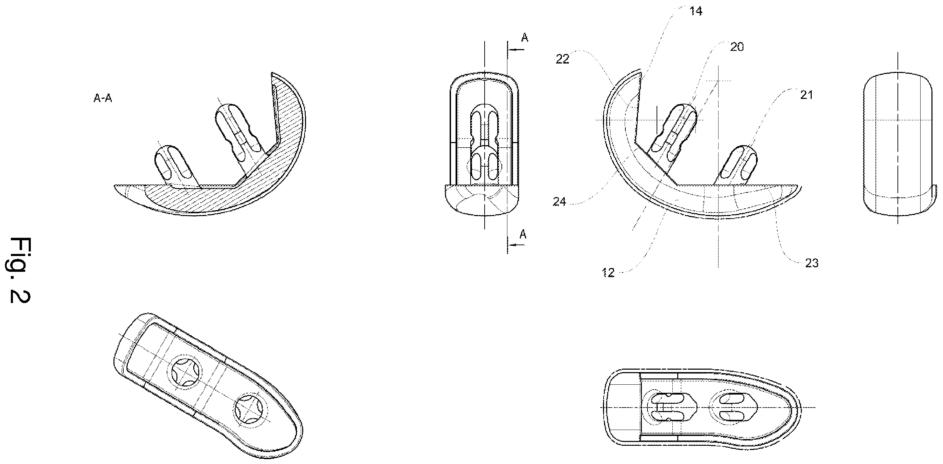

In another preferred embodiment, shown in the appended Figure 2, the first femoral component 10, known as 3CUT, has a curved main body 12 with an inner side comprising two pins 20, 21 facing in a substantially radial direction. Furthermore, the inner side of said main body 12 comprises a first surface 22 at the rear end 14, a second surface 23 at the distal end 13 and a third surface 24 placed in an intermediate position with respect to said first surface 22 and said second surface 23. Preferably, said first surface 22 and said second surface 23 are inclined by an angle of less than 90° and, in particular, said first surface 22 and said second surface 23 are inclined by an angle equal to 85°.

In another preferred embodiment, said two pins 20, 21 are inclined by an angle comprised between 55° and 65° with respect to the plane of said second surface 23. In particular, said two pins 20, 21 are inclined by an angle equal to 60° with respect to the plane of said second surface 23. Inclinations comprised between 55° and 65° allow the fastening of said first femoral component 10 at the end of a femur to be optimized.

Advantageously, said pins 18, 20, 21 are provided with transversal or longitudinal grooves or surface machinings adapted to promote the cooperation with the bone cement used for fastening the prostheses.

Said first femoral component 10 may be made of cobalt chrome molybdenum alloy (e.g. CoCrMo ISO 5832/4 alloy) and may be made in a version to be cemented or a version not to be cemented. For the cementless version a porous titanium coating can be used (Ti-Growth C) deposited through the so-called Vacuum Plasma Spray technique on which a layer of hydroxyapatite (Osprovit) is superimposed.

Whereas titanium alloy prostheses are hypoallergenic, cobalt chrome molybdenum alloy prostheses are not, therefore, for patients sensitive to metal ions such as nickel and, more rarely, cobalt or chromium, cobalt chrome molybdenum alloy prostheses can be made hypoallergenic for example through a coating made of inert ceramic material such as titanium niobium nitride (TiNbN), perfectly biocompatible.

All the metal joint surfaces are preferably mirror polished.

The first femoral component 10 is preferably made in different sizes or measurements, corresponding to the width of said main body 12. For example, 6 sizes corresponding to widths of said main body 12 can be identified, equal to 15 mm, 16 mm, 17 mm, 18 mm, 19 mm and 20 mm.

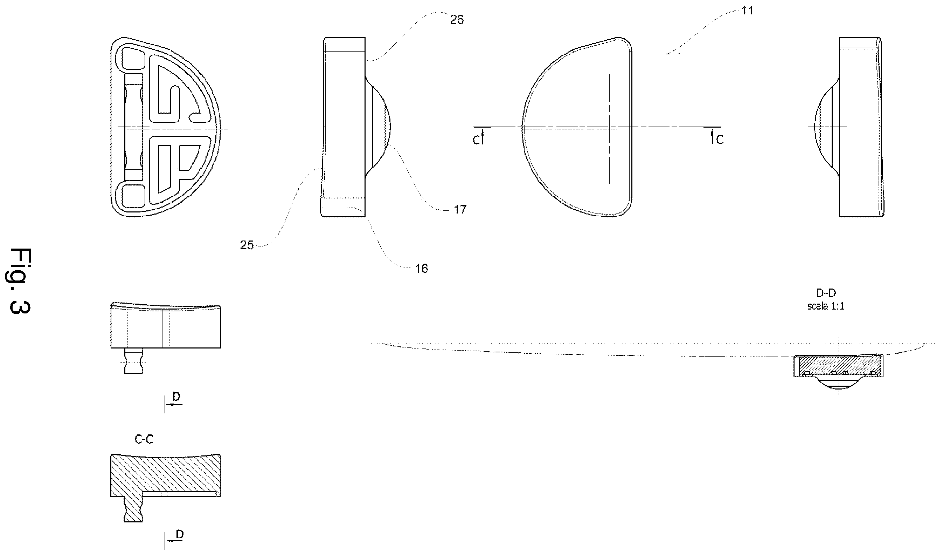

With reference to the appended Figure 3, the second tibial component 11 has a main body 16 of substantially semi-discoidal shape comprising an upper surface 25 adapted to be engaged with the surface of the convex outer side of said first femoral component 10 and a lower surface 26 comprising fastening means at the end of a tibia.

In a preferred embodiment, said upper surface 25 of the second tibial component 11 has a double profile, a first profile with greater congruence in the rear part of said upper surface 25 and a second profile with lower congruence in the front part of said upper surface 25. Preferably, said first and said second profile are of the ellipse arch type. This particular profile creates a light congruence and a partial contact area but allows the coupling of all the femoral measurements of the prosthesis according to the present description with all the tibial ones, without any constraints and guaranteeing complete modularity. Thus the advantages of a larger contact area (which implies a reduction in contact stress of the two main parts of the prosthesis) are combined with the possibility to perfectly adapt the femoral and tibial components to the anatomical characteristics of the individual patient without the usual limitations dictated by the compatibility tables.

In another preferred embodiment of the prosthesis according to the present description, the fastening means at the end of a tibia of said tibial component 11 comprise at least one flap 17 substantially orthogonal to the lower surface 26 of said second tibial component 11. Said flap 17 allows the stabilization of the tibial component on the resected bone and, through the bone cement, the fixation thereof. In fact, the space created in the bone through the relevant broach allows a convenient housing of the flap 17 and the creation of a layer of cement which fastens the tibial component also creating an anti-rotational stabilization. In fact, rotational loads act on the tibial component 11 during walking and the flap 17 opposes such loads, guaranteeing stability. To increase the contact area with the cement, grooves or appropriate machinings can be advantageously performed on the flap 17.

Preferably, the flap 17 of the tibial component 11 according to the present description has a half-moon shape, a height of about 5 mm, a length of about 22 mm and a width of about 4 mm; it is substantially positioned in the center with respect to the anteroposterior direction, whereas, with respect to the mediolateral direction, it is positioned in proximity to the center of the knee.

Advantageously, the lower surface 26 of said second tibial component 11 can have grooves or surface machinings adapted to promote the adhesion of the bone cement and therefore the correct connection of the second tibial component 11 at the end of a tibia. Likewise, also said flap 17 can be shaped so as to promote the stability thereof when in use.

Said second tibial component 11 may, advantageously, be made of polyethylene. In particular, it may be made of linear UHMWPE (Ultra-High-Molecular-Weight PolyEthylene), XLink or cross-linked polyethylene or XLink or cross-linked polyethylene with added Vitamin E. Vitamin E is a powerful antioxidant and has an anti-aging effect. The addition of Vitamin E therefore prevents the oxidation phenomena to which XLink polyethylene is subject and, therefore, reduces the wear thereof. In fact, as well as being the result of the contact stress and shear strain which act between the components of the prosthesis, wear is also caused by the oxidation of the materials used.

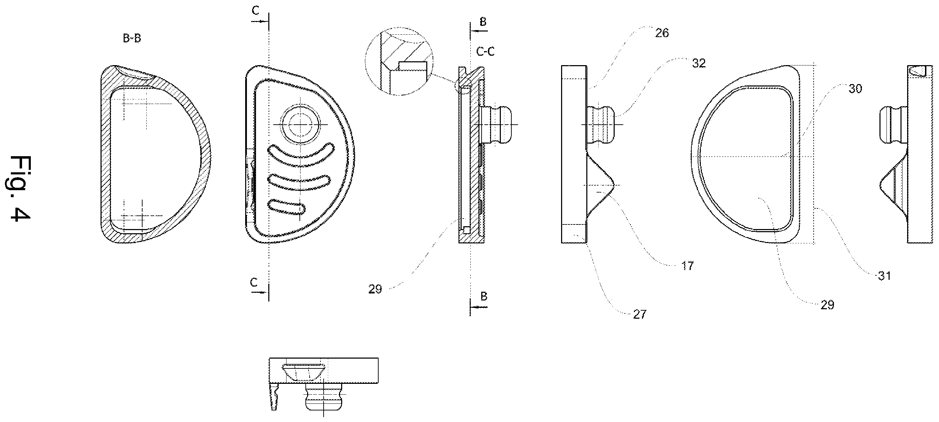

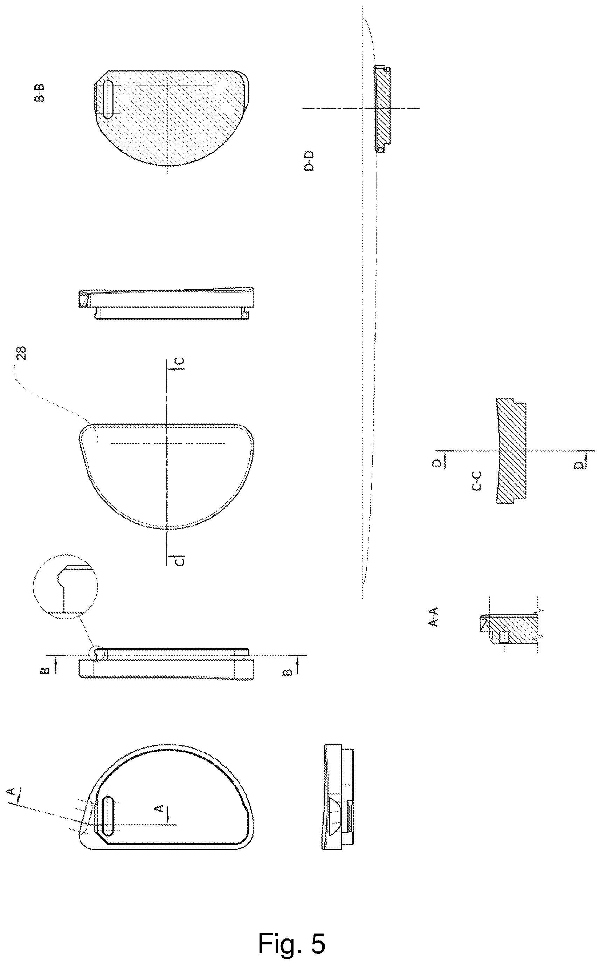

With reference to the preferred embodiment of the present invention illustrated in Fig. 4 and in Fig. 5, said second tibial component 11 comprises two parts, a lower part 27 adapted to interface with the end of a tibia and an insert 28 adapted to interface with the first femoral component 10. Said lower part 27 and said insert 28 of the second tibial component 11 comprise appropriate mutual engagement means. For example, said lower part 27 may comprise a seat 29 adapted to accommodate the insert 28. Advantageously, the edges of said seat 29 and of said insert 28 comprise appropriate mutual locking means made, for example, by one or more protuberances present on the vertical walls of said seat 29, adapted to engage with corresponding grooves present on the outer vertical walls of said insert 28.

In another preferred embodiment of the prosthesis according to the present description, the fastening means at the end of a tibia of said second tibial component 11 comprise at least one flap 17 substantially orthogonal to the lower surface 26 of said second tibial component 11 and a pin 32, also substantially orthogonal to the lower surface 26 of said second tibial component 11. Both the flap 17 and the pin 32 are adapted to be engaged with an end of a tibia in a stable way and such as to promote correct cooperation with the aforesaid femoral component 10 when in place on a patient.

Said pin 32 may further present an axial hole adapted to accommodate a screw adapted to improve the fastening of said second tibial component 11 to the end of a tibia. The use of a screw, for example a cancellous bone screw, is particularly useful in the event that bone cement is not used for fastening the tibial component 11. An example of a cancellous bone screw which may be employed is the Ti6Al4V titanium alloy (ISO 5832/3) screw having diameter 6.5 mm. The screw may be oriented in the hole with an angle of 10°, hence describing a cone with a 20° angle. Furthermore, advantageously, the outer surface of said pin 32 comprises a porous coating adapted to promote the adhesion to the trabacular bone of the tibia. Preferably, said porous coating is made of titanium plasma spray and hydroxyapatite, two materials which promote bone regrowth in their structure and therefore the fastening of the component. In particular, a porous coating of this type with two materials can be deposited on the lower and vertical surface of the flap 17, whereas the pin 32 can be coated with hydroxyapatite only.

In this preferred embodiment, said lower part is preferably made of titanium alloy (e.g. Ti6Al4V alloy ISO 5832/3) or cobalt chrome molybdenum alloy (e.g. CoCrMo alloy ISO 5832/4) and can be made in the version to be cemented and in the version not to be cemented. For the cementless version a porous titanium coating can be used (Ti-Growth C) deposited through the so-called Vacuum Plasma Spray technique on which a layer of hydroxyapatite (Osprovit) is superimposed.

Whereas titanium alloy prostheses are hypoallergenic, cobalt chrome molybdenum alloy prostheses are not, therefore, for patients sensitive to metal ions such as nickel and, more rarely, cobalt or chromium, cobalt chrome molybdenum alloy prostheses can be made hypoallergenic for example through a coating made of inert ceramic material such as titanium niobium nitride (TiNbN), perfectly biocompatible.

Said insert 28 may, instead, be made of polyethylene. In particular, it may be made of linear UHMWPE (Ultra-High-Molecular-Weight PolyEthylene), XLink (or Cross-linked) polyethylene or XLink polyethylene with added Vitamin E.

All the metal joint surfaces are preferably mirror polished.

The second tibial component 11 is preferably made in 6 sizes or measurements corresponding to six different measurements of the dimension 30 in the lateral median plane equal to about 24 mm, 26 mm, 28 mm, 30 mm, 32 mm and 34 mm, which correspond to measurements of the dimension 31 in the anteroposterior median plane equal to about 39 mm, 42 mm, 45 mm, 48 mm, 51 mm and 54 mm. Furthermore, said second tibial component 11 can be advantageously made with different thicknesses, thickness meaning the distance between the upper surface 25 and the lower surface 26. For example, said second tibial component 11 can be advantageously made in four different thicknesses, selected about equal to 8.5 mm, 9.5 mm, 10.5 mm and 12.5 mm.

In the case where the second tibial component 11 comprises two parts, the aforesaid measurements shall be related to the sum of the thicknesses of the lower part 27 and of the insert 28. In this case, in a preferred embodiment of the prosthesis according to the present description, the minimum thicknesses of the four versions of the insert 28 shall be for example equal to about 5.5 mm, 6.5 mm, 7.5 mm and 9.5 mm which, associated with a lower part 27 with a thickness equal to about 3 mm, reach a total thickness of the second tibial component 11 about equal to 8.5 mm, 9.5 mm, 10.5 mm and 12.5 mm.

Because of the anatomy of the tibial plateau which substantially appears to comprise two halves, an inner one (known as medial) and an outer one (known as lateral), the second tibial components 11 are preferably made in two specular versions adapted to be positioned on each of the two halves, the medial half and the lateral half. It is clear that a tibial component 11 adapted to be positioned on the right medial half (RM) will also be adapted to be positioned on the left lateral half (LL). Vice versa, a tibial component 11 adapted to be positioned on the left medial half (LM) will also be adapted to be positioned on the right lateral half (RL). Said tibial components 11 can therefore be advantageously made in two specular versions, indicated respectively as RM/LL and LM/RL on the basis of their destination with respect to the two halves of the head of the tibia.

Correspondingly, also the first femoral components 10 are preferably made in two specular versions adapted to be positioned on each of the two femoral condyles, the medial condyle and the lateral condyle. A femoral component 10 adapted to be positioned on the right medial condyle (RM) will also be adapted to be positioned on the left lateral condyle (LL) in this case too. Vice versa, a femoral component 10 adapted to be positioned on the left medial condyle (LM) will also be adapted to be positioned on the right lateral condyle (RL). Said femoral components 10 can therefore be advantageously made in two specular versions, indicated respectively as RM/LL and LM/RL on the basis of their destination with respect to the two condyles of the femur.

The prosthesis according to the present description introduces a very high degree of flexibility as it is fully compatible and interfaceable between femoral and tibial components made in various sizes and dimensions. The femoral component described is characterized by reduced dimensions and is compatible with the tibial component regardless of the size, also thanks to the double ellipse profile geometry of the contact surface of the tibial component, which identifies areas with differentiated congruence.

The prosthesis according to the invention allows the operating surgeon to choose intraoperatively, during the unicompartmental knee prosthesis operation, which type of femoral component to use between 3CUT and RES, also after having performed all the tibial preparation.

Given the aforesaid flexibility and intraoperativity, the prosthesis according to the present description is suitable to be provided also as a kit comprising all the variations and measurements of the femoral component 10 and of the tibial component 11. In a particular embodiment said kit can therefore comprise:
at least a first femoral component 10, in the version known as 3CUT, for each size of the six sizes corresponding to the widths of the main body 12 of said first femoral component 10 equal to 15mm, 16mm, 17mm, 18mm, 19mm, and 20mm;
at least a first femoral component 10, in the version known as RES, for each size of the six sizes corresponding to the widths of the main body 12 of said first femoral component 10 equal to 15mm, 16mm, 17mm, 18mm, 19mm, and 20mm;
at least a second tibial component 11, in the version known as Allpoly, for each size of the six sizes corresponding to dimensions 30 in the mediolateral plane equal to about 24 mm, 26 mm, 28 mm, 30 mm, 32 mm, and 34 mm, for each overall thickness equal to about 8.5 mm, 9.5 mm, 10.5 mm and 12.5 mm and for each RM/LL and LM/RL type;
at least one lower part 27 of the second tibial component 11 in the version known as Metalback, for each of the six sizes corresponding to dimensions 30 in the lateral medial plane equal to about 24 mm, 26 mm, 28 mm, 30 mm, 32 mm and 34 mm and for each RM/LL and LM/RL type, each preferably comprising at least one cancellous bone screw;
at least one insert 28 for each thickness equal to about 5.5 mm, 6.5 mm, 7.5 mm and 9.5 mm and for each RM/LL and LM/RL type.

Advantageously, said kit can be made in the versions with femoral components 10 and tibial components 11 of the RM/LL and/or LM/RL type.

Furthermore, advantageously, said kit can be made in the versions in which said at least one first femoral component 10 - in the version known as 3CUT and in the version known as RES - and said at least one lower part 27 of the second tibial component 11 in the version known as Metalback, are made in the version to be cemented, in the version not to be cemented, in the hypoallergenic version or in all three versions.

Furthermore, advantageously, said kit can be made in the versions in which said at least one second tibial component 11, in the version known as Allpoly, and said at least one insert 28 are made of linear UHMWPE polyethylene, XLink (cross-linked) polyethylene or XLink polyethylene with added Vitamin E.

## Claims

1. A unicompartmental prosthesis for knee arthroplasty comprising:
a first femoral component (10) having a curved main body (12) with a distal end (13) and a rear end (14), a convex outer side and an inner side comprising fastening means at the end of a femur;
a second tibial component (11) having a single main body (16) of substantially semi-discoidal shape comprising an upper surface (25) adapted to be engaged with the surface of the convex outer side of said first femoral component (10) and a lower surface (26) comprising fastening means at the end of a tibia;
said fastening means at the end of a femur comprising at least one pin (18);
the maximum thickness (15) of the main body (12) of said first femoral component (10) being less than or equal to 7 mm,
**characterized in that** the upper surface (25) of said second tibial component (11) has a double profile, a first profile with higher congruence at the rear part of said second tibial component (11) and a second profile with lower congruence at the front part of said second tibial component (11).

2. A prosthesis according to claim 1, **characterized in that** said first and second profiles are of the ellipse arch type.

3. A prosthesis according to one or more of claims 1 to 2, **characterized in that** said fastening means at the end of a tibia comprise at least one flap (17) substantially orthogonal to the lower surface (26) of said second tibial component (11).

4. A prosthesis according to one or more of claims 1 to 3, **characterized in that** said fastening means at the end of a femur comprise two pins (20, 21).

5. A prosthesis according to claim 4, **characterized in that** the inner side of said main body (12) comprises a first surface (22) at the rear end (14), a second surface (23) at the distal end (13) and a third surface (24) placed in an intermediate position with respect to said first surface (22) and said second surface (23), said first surface (22) and said second surface (23) being inclined at an angle less than 90°.

6. A prosthesis according to claim 5, **characterized in that** said two pins (20, 21) are inclined at an angle between 55° and 65° with respect to the plane of said second surface (23).

7. A prosthesis according to one or more of claims 1 to 3, **characterized in that** said fastening means at the end of a femur further comprise an anti-rotational flap (19).

8. A prosthesis according to claim 7, **characterized in that** the maximum thickness of the main body (12) of said first femoral component (10) is less than or equal to 7 mm at the rear end (14) and less than or equal to 4.5 mm at the distal end (13).

9. A prosthesis according to one or more of claims 1 to 8, **characterized in that** said second tibial component (11) is made of polyethylene.

10. A prosthesis according to one or more of claims 1 to 8, **characterized in that** the main body (16) of said second tibial component (11) comprises a lower part (27) having a lower surface (26) comprising fastening means at the end of a tibia and an insert (28) having an upper surface (25) adapted to interface with the first femoral component (10), said lower part (27) and said insert (28) comprising suitable mutual engagement means.

11. A prosthesis according to claim 10, **characterized in that** said insert (28) is made of polyethylene.

12. A prosthesis according to claims 9 and 11, **characterized in that** said polyethylene is selected from the group consisting of linear UHMWPE polyethylene, XLink polyethylene and XLink polyethylene with added Vitamin E.

13. A prosthesis according to one or more of claims 10 to 12, **characterized in that** said second tibial component (11) comprises a pin (32), substantially orthogonal to the lower surface (26) of the lower part (27) of said second tibial component (11).

14. A prosthesis according to claim 13, **characterized in that** said pin (32) has an axial hole adapted to house a screw adapted to improve the fastening of the lower part (27) of said second tibial component (11) to the end of a tibia.

15. A prosthesis according to one or more of claims 1 to 14, **characterized in that** the flap (17), the pin (32) and the lower surface (26) of said second tibial component (11) and the pins (18, 20, 21) of said first femoral component (10) are provided with transversal or longitudinal grooves or surface machinings adapted to promote cooperation with bone cement.

16. A prosthesis according to one or more of claims 1 to 15, **characterized in that** said first femoral component (10) is made of cobalt chrome molybdenum alloy.

17. A prosthesis according to one or more of claims 1 to 16, **characterized in that** the lower part (27) of the second tibial component (11) is made of titanium alloy or cobalt chrome molybdenum alloy.

18. A kit of unicompartmental prosthesis for knee arthroplasty, comprising: a unicompartmental prosthesis according to any of claims 1-17, and
at least a first femoral component (10) comprising two pins (20, 21), for each size of the six sizes corresponding to the widths of the main body (12) of said first femoral component (10) equal to 15mm, 16mm, 17mm, 18mm, 19mm, and 20mm;
at least a first femoral component (10) comprising a pin (18) and an anti-rotational flap (19), for each size of the six sizes corresponding to the widths of the main body (12) of said first femoral component (10) equal to 15mm, 16mm, 17mm, 18mm, 19mm, and 20mm;
at least a second tibial component (11) comprising a single main body (16) equipped with at least one flap (17), for each size of the six sizes corresponding to a dimension (30) in the mediolateral plane equal to about 24mm, 26mm, 28mm, 30mm, 32mm, and 34mm, and for each overall thickness equal to about 8.5mm, 9.5mm, 10.5mm, and 12.5mm;
at least a second tibial component (11) comprising a lower part (27) about 3mm thick and equipped with at least one flap (17), a pin (32) and a screw, for each size of the six sizes corresponding to a dimension (30) in the mediolateral plane equal to about 24mm, 26mm, 28mm, 30mm, 32mm, and 34mm, and an insert (28) for each thickness equal to about 5.5mm, 6.5mm, 7.5mm, and 9.5mm.

19. A kit according to claim 18, wherein said at least a first femoral component (10) and said at least a second tibial component (11) are of the RM/LL type or of the LM/RL type or of both the aforesaid types.

## Patentansprüche

1. Unikompartimentelle Prothese für Kniearthroplastik, umfassend:
eine erste Oberschenkel-Komponente (10) aufweisend einen gebogenen Hauptkörper (12) mit einem distalen Ende (13) und einem hinteren Ende (14), eine konvexe Außenseite und eine Innenseite, die Befestigungsmittel am Ende eines Oberschenkels umfasst;
eine zweite Schienbein-Komponente (11) aufweisend einen einzigen Hauptkörper (16) mit einer im Wesentlichen halbscheibenförmigen Gestalt umfassend eine Oberseite (25), die so angepasst ist, dass sie mit der Oberfläche der konvexen Außenseite der ersten Oberschenkel-Komponente (10) in Eingriff gebracht werden kann, und eine Unterseite (26), die Befestigungsmittel am Ende eines Schienbeins umfasst;
wobei die Befestigungsmittel am Ende eines Oberschenkels mindestens einen Bolzen (18) umfassen; wobei die maximale Dicke (15) des Hauptkörpers (12) der ersten Oberschenkel-Komponente (10) weniger als oder gleich zu 7 mm beträgt,
**dadurch gekennzeichnet, dass** die Oberseite (25) der zweiten Schienbein-Komponente (11) ein Doppelprofil aufweist, ein erstes Profil mit höherer Übereinstimmung am Hinterteil der zweiten Schienbein-Komponente (11) und ein zweites Profil mit niedrigerer Übereinstimmung am Vorderteil der zweiten Schienbein-Komponente (11).

2. Prothese nach Anspruch 1, **dadurch gekennzeichnet, dass** das erste und das zweite Profil ellipsenbogenartig sind.

3. Prothese nach einem oder mehreren der Ansprüche 1 bis 2, **dadurch gekennzeichnet, dass** die Befestigungsmittel am Ende eines Schienbeins mindestens eine Klappe (17) umfassen, die sich im Wesentlichen orthogonal zur Unterseite (26) der zweiten Schienbein-Komponente (11) erstreckt.

4. Prothese nach einem oder mehreren der Ansprüche 1 bis 3, **dadurch gekennzeichnet, dass** die Befestigungsmittel am Ende eines Oberschenkels zwei Bolzen (20, 21) umfassen.

5. Prothese nach Anspruch 4, **dadurch gekennzeichnet, dass** die Innenseite des Hauptkörpers (12) eine erste Fläche (22) am hinteren Ende (14), eine zweite Fläche (23) am distalen Ende (13) und eine dritte Fläche (24) umfasst, die in einer Zwischenposition in Bezug auf die erste Fläche (22) und die zweite Fläche (23) angeordnet ist, wobei die erste Fläche (22) und die zweite Fläche (23) in einem Winkel von weniger als 90° geneigt sind.

6. Prothese nach Anspruch 5, **dadurch gekennzeichnet, dass** die beiden Bolzen (20, 21) in einem Winkel zwischen 55° und 65° in Bezug auf die Ebene der zweiten Fläche (23) geneigt sind.

7. Prothese nach einem oder mehreren der Ansprüche 1 bis 3, **dadurch gekennzeichnet, dass** die Befestigungsmittel am Ende eines Oberschenkels außerdem eine Gegen-Drehklappe (19) umfassen.

8. Prothese nach Anspruch 7, **dadurch gekennzeichnet, dass** die maximale Dicke des Hauptkörpers (12) der ersten Oberschenkel-Komponente (10) am hinteren Ende (14) weniger als oder gleich zu 7 mm und am distalen Ende (13) weniger als oder gleich zu 4,5 mm beträgt.

9. Prothese nach einem oder mehreren der Ansprüche 1 bis 8, **dadurch gekennzeichnet, dass** die zweite Schienbein-Komponente (11) aus Polyethylen hergestellt wird.

10. Prothese nach einem oder mehreren der Ansprüche 1 bis 8, **dadurch gekennzeichnet, dass** der Hauptkörper (16) der zweiten Schienbein-Komponente (11) einen Unterteil (27) aufweisend eine Unterseite (26) umfassend Befestigungsmittel am Ende eines Schienbeins und einen Einsatz (28) aufweisend eine Oberseite (25) umfasst, die angepasst ist, mit der ersten Oberschenkel-Komponente (10) zusammenzuwirken, wobei der Unterteil (27) und der Einsatz (28) geeignete gegenseitige Eingriffsmittel umfassen.

11. Prothese nach Anspruch 10, **dadurch gekennzeichnet, dass** der Einsatz (28) aus Polyethylen hergestellt wird.

12. Prothese nach einem der Ansprüche 9 und 11, **dadurch gekennzeichnet, dass** das Polyethylen aus der Gruppe bestehend aus linearem UHMWPE, Polyethylen, XLink-Polyethylen und XLink-Polyethylen mit zugesetztem Vitamin E ausgewählt wird.

13. Prothese nach einem oder mehreren der Ansprüche 10 bis 12, **dadurch gekennzeichnet, dass** die zweite Schienbein-Komponente (11) einen Bolzen (32) umfasst, der sich im Wesentlichen orthogonal zur Unterseite (26) des Unterteils (27) der zweiten Schienbein-Komponente (11) erstreckt.

14. Prothese nach Anspruch 13, **dadurch gekennzeichnet, dass** der Bolzen (32) ein Axialbohrung aufweist, die angepasst ist, eine Schraube aufzunehmen, die angepasst ist, die Befestigung des Unterteils (27) der zweiten Schienbein-Komponente (11) am Ende eines Schienbeins zu verbessern.

15. Prothese nach einem oder mehreren der Ansprüche 1 bis 14, **dadurch gekennzeichnet, dass** die Klappe (17), der Bolzen (32) und die Unterseite (26) der zweiten Schienbein-Komponente (11) und die Bolzen (18, 20, 21) der ersten Oberschenkel-Komponente (10) mit Quer-oder-Längsnuten oder Oberflächenbearbeitungen versehen sind, die angepasst sind, das Zusammenwirken mit Knochenzement zu fördern.

16. Prothese nach einem oder mehreren der Ansprüche 1 bis 15, **dadurch gekennzeichnet, dass** die erste Oberschenkel-Komponente (10) aus einer Chrom-Cobalt-Molybdän-Legierung hergestellt wird.

17. Prothese nach einem oder mehreren der Ansprüche 1 bis 16, **dadurch gekennzeichnet, dass** der Unterteil (27) der zweiten Schienbein-Komponente (11) aus einer Titanlegierung oder einer Chrom-Cobalt-Molybdän-Legierung hergestellt wird.

18. Bausatz einer unikompartimentellen Prothese für die Kniearthroplastik, umfassend:
eine unikompartimentelle Prothese nach einem der Ansprüche 1-17 und
mindestens eine erste Oberschenkel-Komponente (10) umfassend zwei Bolzen (20, 21) für jede der sechs Größen, die den Breiten des Hauptkörpers (12) der ersten Oberschenkel-Komponente (10) gleich zu 15mm, 16mm, 17mm, 18mm, 19mm und 20mm entsprechen;
mindestens eine erste Oberschenkel-Komponente (10) umfassend einen Bolzen (18) und eine Gegen-Drehklappe (19) für jede der sechs Größen, die den Breiten des Hauptkörpers (12) der ersten Oberschenkel-Komponente (10) gleich zu 15mm, 16mm, 17mm, 18mm, 19mm und 20mm entsprechen;
mindestens eine zweite Schienbeinkomponente (11) umfassend einen einzigen Hauptkörper (16), der mit mindestens einer Klappe (17) versehen ist, für jede der sechs Größen einer Abmessung (30) in der mediolateralen Ebene gleich zu etwa 24mm, 26mm, 28mm, 30mm, 32mm und 34mm und für jede Gesamtdicke gleich zu etwa 8,5mm, 9,5mm, 10,5mm und 12,5mm entspricht;
mindestens eine zweite Schienbein-Komponente (11) umfassend einen Unterteil (27) mit einer Dicke von etwa 3mm und mit mindestens einer Klappe (17), einem Bolzen (32) und einer Schraube versehen ist, wobei jede der sechs Größen einer Abmessung (30) in der mediolateralen Ebene gleich zu etwa 24mm, 26mm, 28mm, 30mm, 32mm und 34mm entspricht, und einen Einsatz (28) für jede Dicke gleich zu etwa 5,5mm, 6,5mm, 7,5mm und 9,5mm.

19. Bausatz nach Anspruch 18, wobei die mindestens eine erste Oberschenkel-Komponente (10) und die mindestens eine zweite Schienbein-Komponente (11) des RM/LL-Typs oder des LM/RL-Typs oder von beiden der vorgenannten Typen sind.

## Revendications

1. Prothèse unicompartimentale pour l'arthroplastie du genou comprenant :
un premier composant fémoral (10) ayant un corps principal incurvé (12) avec une extrémité distale (13) et une extrémité arrière (14), un côté extérieur convexe et un côté intérieur comprenant des moyens de fixation à l'extrémité d'un fémur ;
un second composant tibial (11) ayant un seul corps principal (16) de forme sensiblement semi-discoïdale comprenant une surface supérieure (25) adaptée pour être engagée avec la surface de la face externe convexe dudit premier composant fémoral (10) et une surface inférieure (26) comprenant des moyens de fixation à l'extrémité d'un tibia ;
lesdits moyens de fixation à l'extrémité d'un fémur comprenant au moins une broche (18) ; l'épaisseur maximale (15) du corps principal (12) dudit premier composant fémoral (10) étant inférieure ou égale à 7 mm,
**caractérisée en ce que** la surface supérieure (25) dudit second composant tibial (11) a un double profil, un premier profil avec une congruence plus élevée au niveau de la partie arrière dudit second composant tibial (11) et un second profil avec une congruence plus faible au niveau de la partie avant dudit second composant tibial (11).

2. Prothèse selon la revendication 1, **caractérisée en ce que** lesdits premier et second profils sont du type arc d'ellipse.

3. Prothèse selon une ou plusieurs des revendications 1 à 2, **caractérisée en ce que** lesdits moyens de fixation à l'extrémité d'un tibia comprennent au moins un rabat (17) sensiblement orthogonal à la surface inférieure (26) dudit second composant tibial (11).

4. Prothèse selon une ou plusieurs des revendications 1 à 3, **caractérisée en ce que** lesdits moyens de fixation à l'extrémité d'un fémur comprennent deux broches (20, 21).

5. Prothèse selon la revendication 4, **caractérisée en ce que** la face interne dudit corps principal (12) comprend une première surface (22) à l'extrémité arrière (14), une deuxième surface (23) à l'extrémité distale (13) et une troisième surface (24) placée dans une position intermédiaire par rapport à ladite première surface (22) et ladite deuxième surface (23), ladite première surface (22) et ladite deuxième surface (23) étant inclinées d'un angle inférieur à 90°.

6. Prothèse selon la revendication 5, **caractérisée en ce que** lesdites deux broches (20, 21) sont inclinées d'un angle compris entre 55° et 65° par rapport au plan de ladite deuxième surface (23).

7. Prothèse selon une ou plusieurs des revendications 1 à 3, **caractérisée en ce que** lesdits moyens de fixation à l'extrémité d'un fémur comprennent en outre un rabat anti-rotation (19).

8. Prothèse selon la revendication 7, **caractérisée en ce que** l'épaisseur maximale du corps principal (12) dudit premier composant fémoral (10) est inférieure ou égale à 7 mm à l'extrémité arrière (14) et inférieure ou égale à 4,5 mm à l'extrémité distale (13).

9. Prothèse selon une ou plusieurs des revendications 1 à 8, **caractérisée en ce que** ledit second composant tibial (11) est en polyéthylène.

10. Prothèse selon une ou plusieurs des revendications 1 à 8, **caractérisée en ce que** le corps principal (16) dudit second composant tibial (11) comprend une partie inférieure (27) ayant une surface inférieure (26) comprenant des moyens de fixation à l'extrémité d'un tibia et un insert (28) ayant une surface supérieure (25) adaptée à l'interface avec le premier composant fémoral (10), ladite partie inférieure (27) et ledit insert (28) comprenant des moyens d'engagement mutuel appropriés.

11. Prothèse selon la revendication 10, **caractérisée en ce que** ledit insert (28) est en polyéthylène.

12. Prothèse selon les revendications 9 et 11, **caractérisée en ce que** ledit polyéthylène est choisi dans le groupe constitué par le polyéthylène UHMWPE linéaire, le polyéthylène XLink et le polyéthylène XLink additionné de vitamine E.

13. Prothèse selon une ou plusieurs des revendications 10 à 12, **caractérisée en ce que** ledit second composant tibial (11) comprend une broche (32), sensiblement orthogonale à la surface inférieure (26) de la partie inférieure (27) dudit second composant tibial (11).

14. Prothèse selon la revendication 13, **caractérisée en ce que** ladite broche (32) a un trou axial adapté pour loger une vis adaptée pour améliorer la fixation de la partie inférieure (27) dudit second composant tibial (11) à l'extrémité d'un tibia.

15. Prothèse selon une ou plusieurs des revendications 1 à 14, **caractérisée en ce que** le rabat (17), la broche (32) et la surface inférieure (26) dudit second composant tibial (11) et les broches (18, 20, 21) dudit premier composant fémoral (10) sont pourvus de rainures transversales ou longitudinales ou d'usinages de surface adaptés pour favoriser la coopération avec le ciment osseux.

16. Prothèse selon une ou plusieurs des revendications 1 à 15, **caractérisée en ce que** ledit premier composant fémoral (10) est en alliage cobalt chrome molybdène.

17. Prothèse selon une ou plusieurs des revendications 1 à 16, **caractérisée en ce que** la partie inférieure (27) du second composant tibial (11) est en alliage de titane ou en alliage de cobalt-chrome-molybdène.

18. Kit de prothèse unicompartimentale pour l'arthroplastie du genou, comprenant :
une prothèse unicompartimentale selon l'une quelconque des revendications 1 à 17, et
au moins un premier composant fémoral (10) comprenant deux broches (20, 21), pour chacune des six tailles correspondant aux largeurs du corps principal (12) dudit premier composant fémoral (10) égales à 15 mm, 16 mm, 17 mm, 18 mm, 19 mm, et 20 mm ;
au moins un premier composant fémoral (10) comprenant une broche (18) et un rabat anti-rotation (19), pour chacune des six tailles correspondant aux largeurs du corps principal (12) dudit premier composant fémoral (10) égales à 15 mm, 16 mm, 17 mm, 18 mm, 19 mm, et 20 mm ;
au moins un second composant tibial (11) comprenant un seul corps principal (16) équipé d'au moins un rabat (17), pour chaque taille des six tailles correspondant à une dimension (30) dans le plan médiolatéral égale à environ 24 mm, 26 mm, 28 mm, 30 mm, 32 mm et 34 mm, et pour chaque épaisseur totale égale à environ 8,5 mm, 9,5 mm, 10,5 mm et 12,5 mm ;
au moins un second composant tibial (11) comprenant une partie inférieure (27) d'environ 3 mm d'épaisseur et équipée d'au moins un rabat (17), d'une broche (32) et d'une vis, pour chacune des six tailles correspondant à une dimension (30) dans le plan médiolatéral égale à environ 24 mm, 26 mm, 28 mm, 30 mm, 32 mm et 34 mm, et un insert (28) pour chaque épaisseur égale à environ 5,5 mm, 6,5 mm, 7,5 mm et 9,5 mm.

19. Kit selon la revendication 18, dans lequel ledit au moins un premier composant fémoral (10) et ledit au moins un second composant tibial (11) sont du type RM/LL ou du type LM/RL ou des deux types susmentionnés.
